Europäisches Patentamt

European Patent Office

Office européen des brevets

(19)

(11) Numéro de publication: **0 215 878
B1**

(12) **FASCICULE DE BREVET EUROPEEN**

(45) Date de publication du fascicule du brevet:
**08.11.89**

(21) Numéro de dépôt: **86901915.8**

(22) Date de dépôt: **01.04.86**

(86) Numéro de dépôt international:
**PCT/FR 86/00109**

(87) Numéro de publication internationale:
**WO 86/05676 (09.10.86** Gazette 86/22)

(51) Int. Cl.⁴: **A 61 B 17/41,** B 26 B 19/00,
A 45 D 26/00

(54) **PROCEDE ET APPAREIL DE RASAGE DE LA BARBE.**

(30) Priorité: **29.03.85 FR 8504825
12.06.85 FR 8508860**

(43) Date de publication de la demande:
**01.04.87 Bulletin 87/14**

(45) Mention de la délivrance du brevet:
**08.11.89 Bulletin 89/45**

(84) Etats contractants désignés:
**CH DE GB IT LI NL**

(56) Documents cités:
**DE-A- 3 220 962
FR-A- 1 560 498
FR-A- 2 526 302
FR-E- 96 443
GB-A- 2 123 287
US-A- 3 197 612
US-A- 3 659 613
US-A- 3 693 623
US-A- 4 388 924**

(73) Titulaire: **POLITZER, Eugène Jim, 65 Rue Jouffroy,
F-75017 Paris (FR)**

(72) Inventeur: **POLITZER, Eugène Jim, 65 Rue Jouffroy,
F-75017 Paris (FR)**

(74) Mandataire: **Viard, Jean, Cabinet VIARD 28 bis, avenue
Mozart, F-75016 Paris (FR)**

ACTORUM AG

## Description

La présente invention a pour objet un procédé de rasage et un appareil permettant la mise en oeuvre du procédé.

Depuis les temps les plus reculés, l'homme a utilisé pour éliminer les poils hors de l'épiderme tout d'abord un couteau en bois ou en os, puis une lame en métal à sec ou avec des savons ou autres produits. Des appareils électriques ont ensuite été conçus, utilisant des lames tournantes ou vibrantes placées sous une grille à ouvertures micrométriques permettant le rasage à sec. On a cherché à améliorer ces moyens par diverses voies, mais les résultats sont néanmoins le plus souvent décevants.

Il se produit en effet une limitation de l'action de rasage par l'impossibilité d'aller avec les lames au-delà du plan de l'épiderme et, en conséquence, en quelques heures les poils repoussent et deviennent visibles de sorte qu'il y a très souvent nécessité d'effectuer un second rasage en fin de journée, notamment chez les personnes à pilosité brune.

Il a déjà été proposé dans la demande de brevet GB-A-2 123 287 et dans US-A-3 693 623 un dispositif d'épilation incluant un générateur de rayon laser du type de ceux qui ont été développés pour les applications médicales, dont le faisceau est guidé par des fibres optiques flexibles, pour être dirigé sur la racine du poil. Cette technique est longue et constitue une incompatibilité fondamentale avec le rasage de la barbe qui nécessite pratiquement qu'un ensemble de poils soient rapidement et simultanément éliminés, en un temps limité. En effet, selon la technique d'épilation précitée, chaque racine de poil doit être traitée individuellement. De plus, la transmission d'un faisceau laser par des fibres optiques risque de poser des problèmes pour des applications domestiques.

Dans US-A-3 197 612 est décrit un rasoir électrique permettant d'éliminer les poils sans moyens de coupe. Une plaque est pourvue de fentes à l'intérieur desquelles pénètrent les poils. Chaque fente est entourée de deux conducteurs permettant de développer un champ électrique à haute fréquence. Les poils entrant dans une fente sont brûlés instantanément par chauffage par induction.

Selon la présente invention, le procédé de rasage de la barbe consistant à apporter de l'énergie thermique par des radiations électromagnétiques à un ensemble de poils passant à travers une grille en vue de leur élimination, est caractérisé en ce que l'énergie thermique, délivrée par une source laser, dont la puissance de sortie est comprise entre 0,5 et 3 Watts, est appliquée sur l'extrémité des poils pour provoquer leur roussissement sur toute leur hauteur, jusqu'à une faible profondeur de l'épiderme.

Il est d'observation courante que le roussissement d'un poil, par exemple lors de l'opération de brûlage, pratiquée par les coiffeurs de la pointe des cheveux, s'effectue en une fraction de seconde. Contrairement à ce que l'on peut supposer, ce brûlage est pratiquement imperceptible par la peau. On peut constater ce fait en passant rapidement la flamme d'une allumette tangentiellement près du dos d'une main ou d'un bras. Les poils se consument jusqu'à l'intérieur des pores sans que l'épiderme n'éprouve de sensation de brûlure. Plusieurs heures s'écoulent ensuite avant que les poils réapparaissent au niveau de l'épiderme, c'est-à-dire exactement dans la position d'un rasage venant d'être fait par lame. L'élimination du poil par combustion est plus profonde que l'élimination par rasage du poil au niveau de la peau car le roussissement remonte dans les pores ce qui permet de réduire d'environ deux fois la fréquence des rasages, étant donné que la lame ne peut agir au delà du plan de l'épiderme. En outre, le rasage par roussissement est comparable à un mouvement d'effaçage bien plus rapide que les procédés classiques actuels de rasage. Efin, la nécessité de remplacer les lames usées des appareils actuels est supprimée.

Le procédé de rasage de la barbe par une méthode sans lame, faisant appel à l'énergie délivrée par une source laser consiste à provoquer un impact de l'énergie lumineuse émise par la source sur les extrêmités supérieures d'un ensemble de poils émergeant de la peau pour provoquer sur ces extrémités un effet thermique de roussissement se répandant sur toute la longueur des poils jusqu'à l'intérieur des pores au-dessous de l'épiderme.

Selon la nouvelle technique du rasage, il est fait usage d'une énergie thermique apportée par le rayonnement lumineux à l'extrémité des poils à raser, d'une manière parfaitement limitée, en utilisant un rayon laser dont les paramètres d'utilisation, la puissance et la durée des tirs sont étudiés en fonction de la distance avec la surface de l'épiderme. Ce procédé, en faisant usage des propriétés des rayons laser, notamment la dessication, la combustion, la volatilisation, la pulvérisation instantanées permet l'élimination des poils par roussissement jusqu'à une profondeur faible de l'épiderme à l'intérieur des pores.

La distance entre l'impact du faisceau laser et la peau est déterminée par le positionnement d'une grille micrométrique à travers laquelle passent les poils à éliminer, déterminant ainsi une largeur de rasage de quelques centimètres par un balayage approprié du faisceau.

Conformément à l'invention, on utilise une source émettrice de rayons laser (laser $CO_2$, laser excimer) comportant des dispositifs de réglage de la température par le réglage de la puissance utilisée en liaison avec une tête de rasage.

Suivant une autre caractéristique de l'invention, on utilise un rayonnement à partir d'un laser Hélium-Néon.

La présente invention vise également un appareil de rasage pour la mise en oeuvre du procédé caractérisé en ce qu'il comprend un corps muni d'une tête de travail comportant un capotage présentant au moins un élément de protection ajouré pour le redressement et l'engagement des poils à l'intérieur de la tête.

D'autres caractéristiques et avantages de l'invention apparaîtront au cours de la description qui va suivre d'un mode particulier de réalisation donné uniquement à titre d'exemple non limitatif en regard des dessins qui représentent:

— la figure 1, une vue en perspective d'un appareil comportant une tête de rasage laser continue;

— la figure 2, représente un rasoir réalisé selon l'invention.

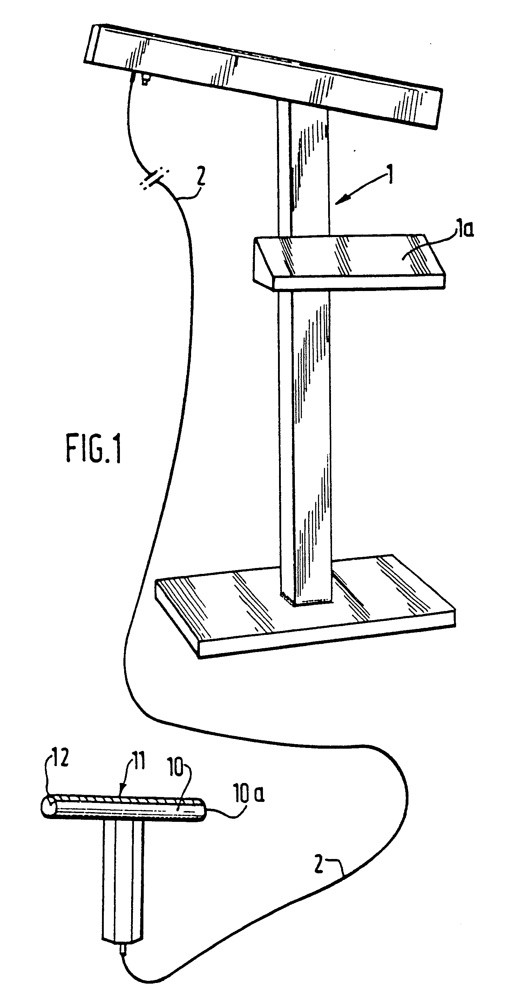

Sur la figure 1, qui représente un dispositif selon le premier mode de réalisation, on voit une tête de rasage par faisceau lumineux laser pouvant être reliée par un cordon souple à une alimentation radio-fréquence (RF).

La figure 1 est une vue en perspective d'un appareil comportant une tête de rasage laser continue ou pouvant être interrompue par l'utilisation d'un volet d'obstruction animé d'un mouvement rotatif ou d'un mouvement de va-et-vient ou de tout système de balayage évitant ainsi l'élévation de la température, ces systèmes pouvant éventuellement conduire à modifier les puissances prévues initialement, par exemple en agissant sur la focalisation.

Le dispositif de rasage est constitué par un ensemble 1, permettant une alimentation radio-fréquence (RF) qui est liée par un cordon souple 2 à une tête de rasage 10 à laser miniaturisée, et donc une pièce qui est tenue à la main par l'utilisateur.

On voit également sur la figure 1 une tête de rasage 10 en forme de T, présentant le long d'une de ses génératrices 10a, une fente 11 permettant le passage d'un rayonnement laser de quelques centimètres de largeur.

Une telle tête 10, qui forme le bloc laser, peut être, d'une manière connue en soi, refroidie par un dispositif de refroidissement (à ailettes ou turbines ou autres), une grille à maille micrométrique en matière isolante 12 (par exemple en Capton) étant interposée entre la peau et l'effet laser sur les extrémités des poils.

Cette tête de rasage exploite une propagation lumineuse guidée dans la tête 10 avec un système de balayage approprié, formant ainsi un guide laser facilement manoeuvrable même par un non-spécialiste qui peut ainsi guider un rayonnement de faible puissance muni d'un dispositif adéquat pour éviter un échauffement de la peau et un risque éventuel pour les yeux. Le capot du rasoir constitue dans ces conditions un masque évitant l'émergence des rayons laser. Avantageusement, on prévoit sur le capot un contact de sécurité inhibant l'émission du faisceau laser tant que le capot n'est pas en place.

Il y a lieu de souligner que le faisceau lumineux peut être à émission continue ou intermittente, statique ou mobile à l'aide d'un écran rotatif placé dans la tête ou par un écran animé d'un mouvement de va-et-vient. Ces différents mouvements sont obtenus le plus souvent à l'aide d'un micro-moteur électrique et d'une transmission mécanique ou magnétique de très faible puissance.

L'équipement permettant l'alimentation radio-fréquednce RF de la tête de laser est monté soit sur un support fixe 1, soit au contraire sur un support mobile pouvant être déplacé aisément à partir de l'alimentation secteur qui est le plus souvent une alimentation 220 Volts/250 Volts de courant alternatif qui est ensuite redressé par un redresseur statique. Un pupître « 1a » de commande et de réglage permet la mise en/et hors service du rasoir.

On obtient ainsi, en un temps extrêmement court, par application de la tête 10, une élimination parfaite des barbes évitant une nouvelle intervention répétée chez l'homme qui demeure bien rasé pendant une période plus longue que celle des systèmes habituels.

Bien qu'à l'heure actuelle les types de laser définis ci-dessus soient les plus intéressants, puisque compris dans la gamme de puissance nécessaire de 0,5 W jusqu'à 3 W, il est possible d'employer également un laser Hélium-Néon avec une puissance supérieure à celle actuellement disponible avec ce type de laser.

Une miniaturisation peut permettre d'obtenir une alimentation intégrée et indépendante ramenant les dimensions de l'ensemble à celles d'un rasoir courant.

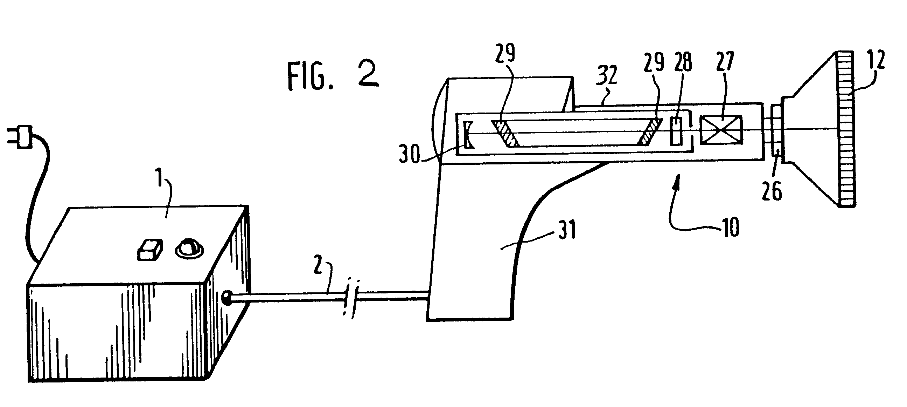

La figure 2 représente un rasoir à laser. Ce rasoir comporte comme décrit en regard de la figure 1, un bloc d'alimentation 1 relié par un cordon électrique 2 à la tête 10. Celle-ci comprend, comme précédemment, un laser $CO_2$ guide d'onde et une grille 12 en matière plastique du genre CAPTON, montée pivotante en 26, autour de l'axe de la tête laser pour pouvoir suivre les contours du visage. La tête laser 10 se compose d'une poignée 31 solidaire d'un tube 32. La cavité tubulaire définie par le tube 32 comprend une cavité comprenant un gaz, disposée entre deux faces de Brewster 29, l'ensemble étant disposé entre deux miroirs 28 et 30. A la sortie du laser proprement dit, est disposé un dispositif de balayage 27 permettant de faire dériver le faisceau pour qu'il balaie, en un temps très court, la surface de la grille 12, à travers laquelle passent les poils de la barbe à éliminer.

## Revendications

1. Procédé de rasage de la barbe par apport d'énergie thermique à un ensemble de poils passant à travers une grille au moyen d'un rayonnement électromagnétique, caractérisé en ce que l'énergie délivrée par une source laser, dont la puissance émise est comprise entre 0,5 et 3 Watts, est appliquée sur l'extrémité des poils pour que cette énergie se répande sur toute la hauteur des poils en provoquant leur roussissement sur toute leur hauteur, jusqu'à une faible profondeur de l'épiderme, à l'intérieur des pores.

2. Dispositif de mise en oeuvre du procédé de rasage de la barbe selon la revendication 1, comprenant une source d'alimentation à radio-fréquences, reliée par un cordon souple (2) à une tête de rasage (10) incluant une source laser miniaturisée (28, 29, 30), reliée mécaniquement à une grille (12), à travers laquelle passent les poils de la barbe à éliminer.

3. Dispositif selon la revendication 2, caractérisé en ce que la source laser est constituée par un laser à $CO_2$.

## Patentansprüche

1. Verfahren zur Bartrasur durch Anwenden thermischer Energie auf eine Ansammlung von Haaren, die mit Hilfe elektromagnetischer Strahlung durch ein Gitter (Netz) ragen, dadurch gekennzeichnet, daß die von einem Laser, dessen ausgestrahlte Leistung zwischen 0,5 und 3 Watt liegt, stammende Energie so auf das Ende der Haare einwirkt, daß sich diese Energie auf die gesamte Haarlänge ausbreitet und ein Verbrennen der Haare über deren gesamte Länge bis zu einer geringen Hauttiefe im Inneren der Poren hervorgerufen wird.

2. Vorrichtung zum Durchführen des Verfahrens nach Anspruch 1, bestehend aus einer Hochfrequenzquelle, die durch ein Kabel (2) mit einem Rasierkopf (10) verbunden ist, der eine Miniatur-Laserquelle (28, 29, 30) enthält und mechanisch mit einem Gitter (Netz) (12) verbunden ist, durch das die zu entfernenden Barthaare ragen.

3. Vorrichtung gemäß Anspruch 2, dadurch gekennzeichnet, daß die Laserquelle aus einem $CO_2$-Laser besteht.

**Claims**

1. A method of shaving the beard by conveying thermal energy by means of electromagnetic radiation to a set of hairs passing through a grille, the method being characterized in that the energy delivered by a laser source having an output power lying in the range 0.5 watts to 3 watts, is applied to the ends of the hairs so that said energy spreads along the entire length of the hairs causing them to singe along their entire length, down to a small depth beneath the epidermis, inside the pores.

2. A device for implementing the method of shaving the beard of claim 1, comprising a radiofrequency source connected via a flexible cord (2) to a shaver head (10) including a miniaturized laser source (28, 29, 30), and mechanically connected to a grille (12) through which there pass the hairs of the beard to be removed.

3. A device according to claim 2, characterized in that the laser source is constituted by a $CO_2$ laser.

FIG.1

FIG. 2

EP 0 215 878 B1